# EUROPEAN PATENT APPLICATION

(11) **EP 4 748 432 A2**
(43) Date of publication of application: **27.05.2026**
(21) Application number: 26171912.4
(22) Date of filing: 03.05.2022
(51) Int. Cl.: A61M 39/10

(54) **OVERMOLDED CONNECTORS FOR TUBING**

(30) Priority: 05.05.2021 US 202163184528 P
(62) Divisional of application: 22725062.8
(71) Applicant: CareFusion 303, Inc., San Diego, CA 92130 (US)
(72) Inventor: Suwito, Wantjinarjo, West Linn, 97068 (US); Feith, Raymond P., Chino Hills, 91709 (US); Canizales, Zachary Edward, Oceanside, 92056 (US); Huang, Kuochu Colin, Walnut, 91789 (US)
(74) Representative: Zenz Patentanwälte Partnerschaft mbB

(57) **Abstract**

A method for connecting a tubing assembly (150) to a tubing system component. The tubing assembly comprises a tubing (152) and a tubing assembly connector (158) circumferentially disposed around a portion of the tubing. A portion of the tubing assembly connector is disposed within a component connector (132) of the tubing system component. A support ring (270) is disposed within a lumen (151) of the tubing to structurally support the tubing during subsequent molding. A coupling portion (160) is molded over the tubing assembly connector and the component connector, thereby forming a secure and sealed connection.

## Description

### FIELD OF THE INVENTION

The present disclosure generally relates to connectors, and, in particular, to tubing connectors.

### BACKGROUND

Medical treatments often include the infusion of a medical fluid (e.g., a saline solution or a liquid medication) to patients using an intravenous (IV) catheter that is connected though an arrangement of flexible tubing and fittings, commonly referred to as an "IV set," to a source of fluid, for example, an IV bag. IV sets can include various components, such as needles/catheters, an IV pump, drip chambers, check valves, access ports, luers, and/or clamps for controlling the fluid flow in the IV line. As can be appreciated, each component of the IV set can be connected to each other via a joint or junction.

In some applications, variations between tubing and connectors can prevent sealing and/or mating between joints or junctions.

### SUMMARY

The disclosed subject matter relates to connectors. In certain embodiments, a tubing system is disclosed that comprises a tubing assembly, comprising: a tubing defining a tubing lumen, the tubing having a tubing elastic modulus; and a tubing assembly connector coupled to the tubing, the tubing assembly connector defining a connector lumen in fluid communication with the tubing lumen, and the tubing assembly connector having a tubing assembly connector elastic modulus greater than the tubing elastic modulus; a tubing system component, comprising: a component connector abutted against the tubing assembly connector, the component connector having a component connector elastic modulus greater than the tubing elastic modulus; and a coupling portion overmolded over the tubing assembly connector and the component connector to couple the tubing to the tubing system component.

In certain embodiments, a method is disclosed that comprises abutting a component connector of a tubing system component against a tubing assembly connector of a tubing assembly to permit fluid communication between the tubing system component and the tubing assembly; and overmolding a coupling portion over the tubing assembly connector and the component connector to couple the tubing assembly connector and the component connector.

In certain embodiments, a method is disclosed that comprises abutting a component connector of a tubing system component against a tubing assembly connector of a tubing assembly to permit fluid communication between the tubing system component and the tubing assembly; and ultrasonically welding the component connector and the tubing assembly connector to couple the tubing assembly connector and the component connector.

It is understood that various configurations of the subject technology will become readily apparent to those skilled in the art from the disclosure, wherein various configurations of the subject technology are shown and described by way of illustration. As will be realized, the subject technology is capable of other and different configurations and its several details are capable of modification in various other respects, all without departing from the scope of the subject technology. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
FIG. 1 illustrates a patient receiving an infusion of a medical fluid through an IV pump according to certain aspects of the present disclosure.
FIG. 2 illustrates a perspective view of a Y-body junction, according to certain aspects of the present disclosure.
FIG. 3 illustrates a cross-sectional view of a portion of the Y-body junction of FIG. 2.
FIG. 4 illustrates a front view of a tubing assembly for use with the Y-body junction of FIG. 2.
FIG. 5 illustrates a cross-sectional view of a tubing system, according to certain aspects of the present disclosure.
FIG. 6 illustrates a front view of a check valve assembly, according to certain aspects of the present disclosure.
FIG. 7 illustrates a cross-sectional view of the check valve assembly of FIG. 6.
FIG. 8 illustrates a cross-sectional view of a tubing system, according to certain aspects of the present disclosure.
FIG. 9 illustrates a front view of a tubing assembly for use with the Y-body junction of FIG. 8.

### DETAILED DESCRIPTION

The disclosed tubing system incorporates a coupling portion that is overmolded over connectors within the tubing system. The overmolded coupling portion can rapidly and effectively seal and couple the connectors of the tubing system, to allow for a secure, leak-free connection with less manufacturing complexity.

The detailed description set forth below is intended as a description of various configurations of the subject technology and is not intended to represent the only configurations in which the subject technology may be practiced. The detailed description includes specific details for the purpose of providing a thorough understanding of the subject technology. However, it will be apparent to those skilled in the art that the subject technology may be practiced without these specific details. In some instances, well-known structures and components are shown in block diagram form in order to avoid obscuring the concepts of the subject technology. Like components are labeled with identical element numbers for ease of understanding. Reference numbers may have letter suffixes appended to indicate separate instances of a common element while being referred to generically by the same number without a suffix letter.

While the following description is directed to the connection of medical fittings for the administration of medical fluid using the disclosed connectors, it is to be understood that this description is only an example of usage and does not limit the scope of the claims. Various aspects of the disclosed connectors may be used in any application where it is desirable to provide a secure fluid connection.

The disclosed connectors overcome several challenges discovered with respect to certain conventional connectors. One challenge with certain conventional connectors is that certain conventional connectors require a precision fit between mating surfaces and/or the use of solvent to allow for a secure connection therebetween, leading to connections that may leak and/or separate. Further, solvents may require a significant amount of time to cure (e.g., 24 hours). Additionally, solvents may require the use of translucent materials to allow for ultraviolet light to cure the solvents. Another challenge with certain conventional connectors is that certain conventional connectors may employ an application or size specific design, such as a mating collar and ring. Because leaking or separated connectors can interrupt the administration of medical fluids, the use of solvents or adhesives can be time consuming and difficult to automate, the selection of materials can be limited, and because manufacturing mating connector portions can lead to complexities in testing and manufacturing, the use of certain conventional connectors is undesirable.

Therefore, in accordance with the present disclosure, it is advantageous to provide a connector as described herein that allows imperfections between mating surfaces to permit secure and leak-free connections. Additionally, it is advantageous to provide a connector that can be set or otherwise ready to use or manipulate for other operations in a short period of time. Further, it is advantageous to provide a connector that can be assembled and manipulated by automated processes.

Examples of connectors that allow secure and leak-free connections are now described.

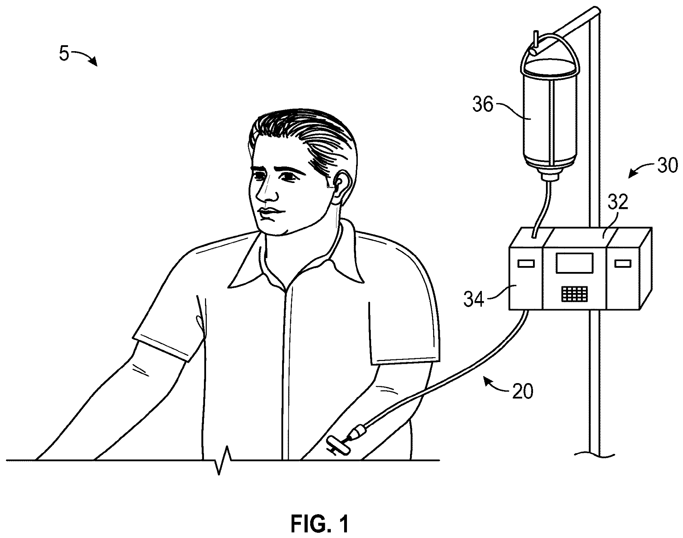

FIG. 1 illustrates a patient 5 receiving an infusion of a medical fluid through an IV pump 30 according to certain aspects of the present disclosure. The IV pump 30 comprises a controller 32 and two pump modules 34. An IV set 20 is connected between a container 36 of the medical fluid and the patient 10. As can be appreciated, the IV set 20 can include various components, such as needles/catheters, drip chambers, check valves, access ports, luers, and/or clamps for controlling the fluid flow in the IV line. As described herein, each component of the IV set can be connected to each other via a connector.

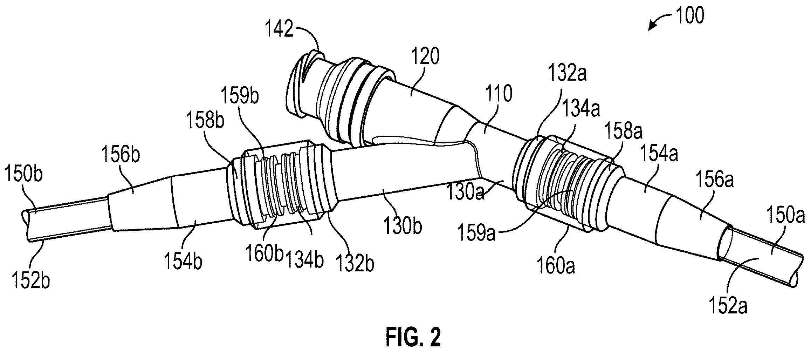

FIG. 2 illustrates a perspective view of a tubing system 100, according to certain aspects of the present disclosure. As illustrated, the tubing system 100 allows for the connection of a first tubing assembly 150a and a second tubing assembly 150b to permit the flow of medical fluid.

As illustrated, a Y-body junction 110 can define a flow path to permit fluid communication between the first tubing assembly 150a and the second tubing assembly 150b. In some embodiments, the Y-body junction 110 can include an injection port 122 to introduce additional flow (e.g. medication) into the fluid flow between the first tubing assembly 150a and the second tubing assembly 150b.

As illustrated, the first tubing assembly 150a can be coupled to a first branch 130a of the Y-body junction 110. The second tubing assembly 150b can be coupled to a second branch 130b of the Y-body junction 110. As can be appreciated, the first branch 130a and the second branch 130b of the Y-body junction 110 can be in fluid communication. Further, the injection port 122 can be coupled to a third branch 120 of the Y-body junction 110. The third branch 120 can be in fluid communication with the first branch 130a and/or the second branch 130b of the Y-body junction 110. As illustrated, the first branch 130a, the second branch 130b, and the third branch 120 can be arranged in a "Y" shaped arrangement.

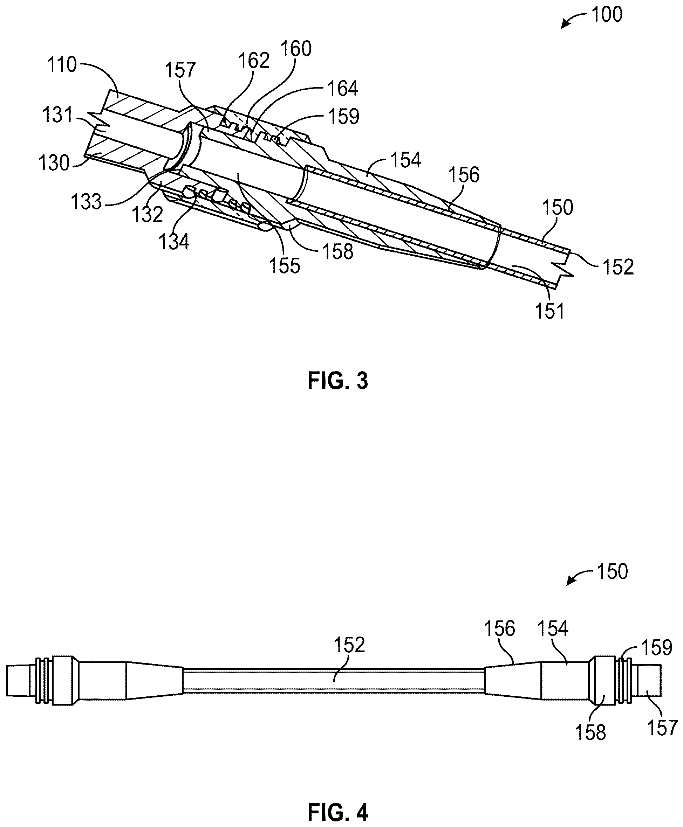

FIG. 3 illustrates a cross-sectional view of a portion of the Y-body junction 110 of FIG. 2. With reference to FIGS. 2 and 3, tubing assemblies 150a, 150b (generally referred to as tubing assembly 150) can be coupled to branches 130a, 130b (generally referred to as branch 130) of the Y-body junction 110. For example, the tubing assembly 150 can be coupled generally to a branch 130 of the Y-body junction 110. As can be appreciated, the configuration and arrangement of the tubing assembly 150 and the branch 130 can apply to the coupling of the first tubing assembly 150a with the first branch 130a and/or the coupling of the second tubing assembly 150b with the second branch 130b.

As illustrated, a termination portion 154 of the tubing assembly 150 can include a connector 158 terminating the tubing 152 of the tubing assembly 150. The connector 158 can be abutted against or otherwise adjacent to a connector 132 formed at an end portion of the branch 130 to provide fluid communication between the tubing lumen 151 of the tubing 152 and the branch lumen 131. In some embodiments, a portion of connector 158, such as the extension portion 157 of the connector 158 can be inserted inside a connector lumen 133 of the connector 132. As can be appreciated, the connector lumen 155 can be in fluid communication with the connector lumen 133.

In the depicted example, the connection between the connector 132 of the Y-body junction 110 and the connector 158 of the tubing assembly 150 can be secured, locked, or otherwise retained by a coupling portion 160 disposed over or around at least a portion of the connector 132 and the connector 158.

As illustrated, the coupling portion 160 engages with at least a portion of the connector 132 and the connector 158. As illustrated, the coupling portion 160 can be circumferentially disposed around at least a portion of the connector 132 and the connector 158. In some embodiments, the coupling portion 160 can include one or more grooves 162 along an inner surface of the coupling portion 160 to engage with ribs 134 extending radially from the connector 132 and/or ribs 159 extending radially from the connector 158. Optionally, the coupling portion 160 can include a center portion 164 configured to align, position, or otherwise engage with the connector 132 and/or the connector 158.

In the depicted example, the coupling portion 160 can be formed by an overmolding process, wherein a material is overmolded over at least a portion of the connector 132 and the connector 158. The coupling portion 160 can be formed from rigid or high elastic modulus materials such as stiff polymers and/or filled polymers, such as glass filled polymers. As can be appreciated, the connector 132 and the connector 158 can be formed from materials that withstand pressure from an overmolding or injection molding process (e.g., 5,000 psi to 12,000 psi) for the coupling portion 160. As described herein, the connector 132 and/or the connector 158 can be formed from rigid or high elastic modulus materials such as stiff polymers and/or filled polymers, such as glass filled polymers.

Optionally, the coupling portion 160 can be stiffer, more rigid, or otherwise have a higher elastic modulus than the connector 132 and/or the connector 158. In some embodiments, the coupling portion 160 can have a similar stiffness or elastic modulus as the connector 132 and/or the connector 158. Further, the coupling portion 160 can be more compliant or otherwise have a lower elastic modulus than the connector 132 and/or the connector 158. As can be appreciated, the coupling portion 160 can be stiffer, more rigid, or otherwise have a higher elastic modulus than the tubing 152.

FIG. 4 illustrates a front view of a tubing assembly 150 for use with the Y-body junction 110 of FIG. 2. With reference to FIGS. 2-4, as described herein, the tubing 152 can be terminated with a termination portion 154 to allow the connection of the tubing 152 and components of the IV set, such as the Y-body junction 110 to allow fluid flow therethrough.

In the depicted example, the termination portion 154 engages with, or is otherwise coupled to a portion of the tubing 152. The termination portion 154 is circumferentially disposed around the portion of the tubing 152. In some embodiments, the termination portion 154 includes a tapered portion 156 to provide strain relief for the tubing 152. As illustrated, the tapered portion 156 can taper away from the connector 158, and taper or reduces in radius away from the connector 158.

In the depicted example, the termination portion 154 can be formed by an overmolding process, wherein a material is overmolded over at least a portion of the tubing 152. The termination portion 154 can be formed from rigid or high elastic modulus materials such as stiff polymers and/or filled polymers, such as glass filled polymers. In the depicted example, the termination portion 154 can be stiffer, more rigid, or otherwise have a higher elastic modulus than the tubing 152.

During the overmolding of the termination portion 154, a core pin or support can be inserted into the lumen 151 of the tubing 152 to allow the tubing 152 to maintain the structure or shape of the tubing 152 or otherwise withstand pressure from an overmolding or injection molding process (e.g., 5,000 psi to 12,000 psi) for the termination portion 154. The core pin can be removed from the lumen 151 after the overmolding of the termination portion 154, allowing flow through the tubing 152 and/or the termination portion 154.

After the overmolding of the termination portion 154, the termination portion 154 withstand pressure from an overmolding or injection molding process (e.g., 5,000 psi to 12,000 psi) for the coupling portion 160 to couple the tubing assembly 150 to a component of the IV set, such as the Y-body junction 110.

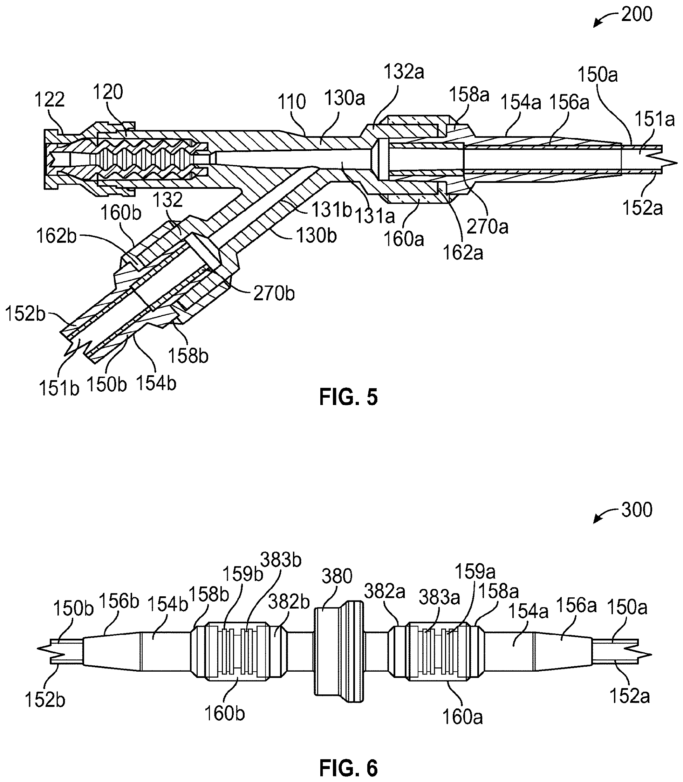

FIG. 5 illustrates a cross-sectional view of a tubing system 200, according to certain aspects of the present disclosure. In some embodiments, the tubing system 200 can include support rings 270a, 270b to support tubing 152a, 152b during the overmolding process of the termination portion 154a, 154b and/or the coupling portion 160a, 160b. As can be appreciated, the support rings 270a, 270b, can allow the tubing 152a, 152b to maintain the structure or shape of the tubing 152a, 152b during an overmolding process. Further, as illustrated, the support rings 270a, 270b can define a lumen therethrough, allowing flow through the tubing 152a, 152b and/or the termination portion 154a, 154b without removing the support rings 270a, 270b. Advantageously, the support rings 270a, 270b allow for the termination portion 154a, 154b to be overmolded onto the tubing 152a, 152b without removing the support rings 270a, 270b after the overmolding process. Further, the support rings 270a, 270b can allow for the overmolding of the coupling portion 160a, 160b after the overmolding of the termination portion 154a, 154b without removing the support rings 270a, 270b.

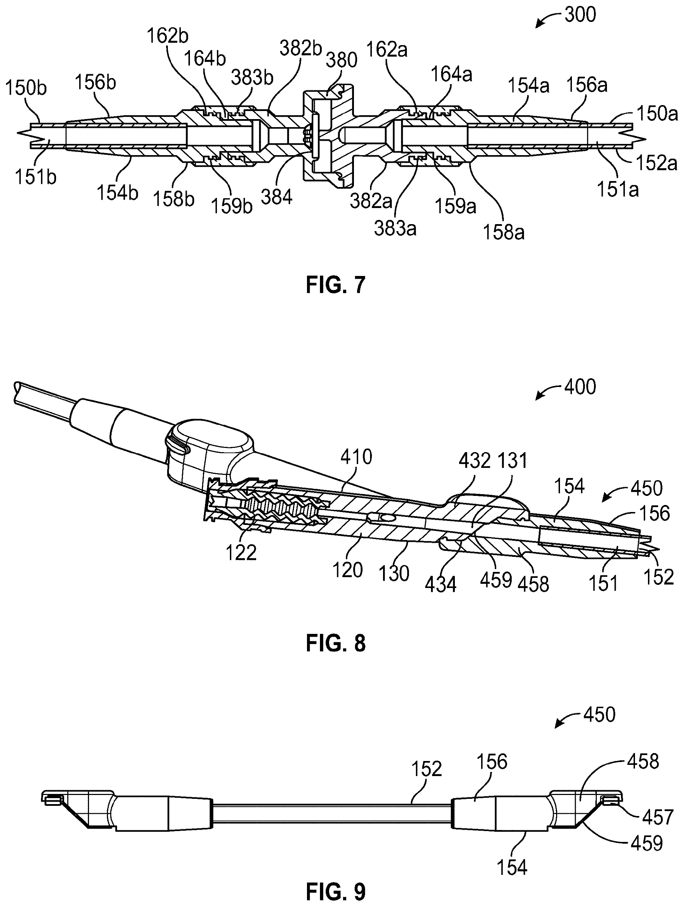

FIG. 6 illustrates a front view of a check valve assembly 300, according to certain aspects of the present disclosure. FIG. 7 illustrates a cross-sectional view of the check valve assembly 300 of FIG. 6. In the depicted example, the check valve assembly 300 can control the direction of flow between the first tubing assembly 150a and the second tubing assembly 150b.

During operation, the check valve 380 can control the direction of flow between the first tubing assembly 150a and the second tubing assembly 150b. The check valve 380 can include a valve element 384 to allow fluid flow to flow in a first direction and prevent flow (e.g., backflow) in a second direction. For example, the check valve 380 may permit flow from the first tubing assembly 150a to the second tubing assembly 150b and prevent flow from the second tubing assembly 150b to the first tubing assembly 150a, or vice versa.

As illustrated, the first tubing assembly 150a can be coupled to a first connector 382a of the check valve 380. Similarly, the second tubing assembly 150b can be coupled to a second connector 382b of the check valve 380. The first connector 382a can be in fluid communication with the second connector 382b across the valve element 384 of the check valve 380.

Similar to the arrangement described with respect to the tubing system 100, the connector 158a can be abutted against or otherwise adjacent to a connector 382a of the check valve 380 to provide fluid communication between the tubing lumen 151a of the tubing 152a and the check valve 380. Further, the connector 158b can be abutted against or otherwise adjacent to a connector 382b of the check valve 380 to provide fluid communication between the tubing lumen 151b of the tubing 152b and the check valve 380.

In the depicted example, the connection between the connector 382a of the check valve 380 and the connector 158a of the tubing assembly 150a can be secured, locked, or otherwise retained by a coupling portion 160a disposed over or around at least a portion of the connector 382a and the connector 158a. Similarly, the connection between the connector 382b of the check valve 380 and the connector 158b of the tubing assembly 150b can be secured, locked, or otherwise retained by a coupling portion 160b disposed over or around at least a portion of the connector 382b and the connector 158b.

As illustrated, the coupling portion 160a,160b engages with at least a portion of the connector 382a, 382b and the connector 158a, 158b. As illustrated, the coupling portion 160a, 160b can be circumferentially disposed around at least a portion of the connector 382a, 382b and the connector 158a, 158b. In some embodiments, the coupling portion 160a, 160b can include one or more grooves 162a, 162b along an inner surface of the coupling portion 160a, 160b to engage with ribs 383a, 383v extending radially from the connector 382a, 382b and/or ribs 159a, 159b extending radially from the connector 158a, 158b. Optionally, the coupling portion 160a, 160b can include a center portion 164a, 164b configured to align, position, or otherwise engage with the connector 382a, 382b and/or the connector 158a, 158b.

In the depicted example, the coupling portion 160a, 160b can be formed by an overmolding process, wherein a material is overmolded over at least a portion of the connector 382a, 382b and the connector 158a, 158b. The coupling portion 160a, 160b can be formed from rigid or high elastic modulus materials such as stiff polymers and/or filled polymers, such as glass filled polymers. As can be appreciated, the connector 382a, 382b and the connector 158a, 158b can be formed from materials that withstand pressure from an overmolding or injection molding process (e.g., 5,000 psi to 12,000 psi) for the coupling portion 160a, 160b. As described herein, the connector 382a, 382b and/or the connector 158a, 158b can be formed from rigid or high elastic modulus materials such as stiff polymers and/or filled polymers, such as glass filled polymers.

FIG. 8 illustrates a cross-sectional view of a tubing system 400, according to certain aspects of the present disclosure. Similar to tubing system 100, the tubing system 400 includes a Y-body junction 410 to direct flow from a tubing assembly 450.

In the depicted example, the tubing assembly 150 can be coupled to a branch 130 of the Y-body junction 410. As illustrated, a termination portion 154 of the tubing assembly 150 can include a connector 458 terminating the tubing 152 of the tubing assembly 150. The connector 458 can be abutted against or otherwise adjacent to a connector 432 formed at an end portion of the branch 130 to provide fluid communication between the tubing lumen 151 of the tubing 152 and the branch lumen 131. In the depicted example, the connector 432 can include a beveled interface 434. The beveled interface 434 can be angled relative to the radial direction of the connector 432. The connector 458 can include a mating beveled interface 459 to abut or engage with the beveled interface 434 at angle relative to the radial direction of the connector 432 and/or the connector 458.

In the depicted example, the connection between the connector 132 of the Y-body junction 410 and the connector 158 of the tubing assembly 150 can be secured or otherwise retained by joining the beveled interface 459 of the connector 458 and the beveled interface 434 of the connector 432. In the depicted example, beveled interface 459 of the connector 458 and the beveled interface 434 of the connector 432 can be joined by ultrasonic welding. In some embodiments, the ultrasonic weld can join the beveled interface 459 of the connector 458 and the beveled interface 434 of the connector 432 by welding in a direction that is perpendicular to the radial direction of the connector 432 and/or the connector 458.

FIG. 9 illustrates a front view of a tubing assembly 450 for use with the Y-body junction 410 of FIG. 8. With reference to FIGS. 8 and 9, the tubing 152 can be terminated with a termination portion 154 to allow the connection of the tubing 152 and components of the IV set, such as the Y-body junction 410 to allow fluid flow therethrough.

As can be appreciated, the termination portion 154 can be formed by an overmolding process similar to the process described with respect to the tubing assembly 150, wherein a material is overmolded over at least a portion of the tubing 152. The termination portion 154 can be formed from rigid or high elastic modulus materials such as stiff polymers and/or filled polymers, such as glass filled polymers. In the depicted example, the termination portion 154 can be stiffer, more rigid, or otherwise have a higher elastic modulus than the tubing 152.

### Illustration of Subject Technology as Clauses

The subject technology is illustrated, for example, according to various aspects described below. Various examples of aspects of the subject technology are described as numbered clauses (1, 2, 3, etc.) for convenience. These are provided as examples and do not limit the subject technology. It is noted that any of the dependent clauses may be combined in any combination, and placed into a respective independent clause, e.g., clause 1 or clause 5. The other clauses can be presented in a similar manner.

Clause 1. A tubing system, comprising: a tubing assembly, comprising: a tubing defining a tubing lumen, the tubing having a tubing elastic modulus; and a tubing assembly connector coupled to the tubing, the tubing assembly connector defining a connector lumen in fluid communication with the tubing lumen, and the tubing assembly connector having a tubing assembly connector elastic modulus greater than the tubing elastic modulus; a tubing system component, comprising: a component connector abutted against the tubing assembly connector, the component connector having a component connector elastic modulus greater than the tubing elastic modulus; and a coupling portion overmolded over the tubing assembly connector and the component connector to couple the tubing to the tubing system component.

Clause 2. The tubing system of Clause 1, wherein the coupling portion is circumferentially disposed around the tubing assembly connector and the component connector.

Clause 3. The tubing system of Clause 1, wherein the tubing assembly connector comprises at least one rib radially extending from the tubing assembly connector.

Clause 4. The tubing system of Clause 3, wherein the coupling portion comprises at least one groove disposed along an inner surface of the coupling portion, wherein the at least one groove engages with the at least one rib of the tubing assembly connector.

Clause 5. The tubing system of Clause 1, wherein the coupling portion comprises a center portion disposed along an inner surface of the coupling portion, wherein the center portion is adjacent to the tubing assembly connector and the component connector.

Clause 6. The tubing system of Clause 1, wherein the tubing system component further comprises: a Y-body junction coupled to the component connector, wherein the Y-body junction is in fluid communication with the tubing assembly.

Clause 7. The tubing system of Clause 6, wherein the Y-body junction comprises an injection site in fluid communication with the tubing assembly.

Clause 8. The tubing system of Clause 6, wherein the Y-body junction comprises a second component connector coupled to the Y-body junction, the second component connector having a second component connector elastic modulus greater than the tubing elastic modulus, the second component connector is abutted against a second tubing assembly, and a second coupling portion is overmolded over a second tubing assembly connector of the second tubing assembly and the second component connector.

Clause 9. The tubing system of Clause 1, wherein the tubing system component further comprises: a check valve coupled to the component connector, wherein the check valve is in fluid communication with the tubing assembly to control the direction of flow through the tubing assembly.

Clause 10. The tubing system of Clause 9, wherein the check valve comprises a second component connector coupled to the check valve, the second component connector having a second component connector elastic modulus greater than the tubing elastic modulus, the second component connector is abutted against a second tubing assembly, and a second coupling portion is overmolded over a second tubing assembly connector of the second tubing assembly and the second component connector.

Clause 11. The tubing system of Clause 1, wherein the tubing assembly connector further comprises a tapered portion extending away from the coupling portion, and the tapered portion tapers in radius away from the coupling portion.

Clause 12. The tubing system of Clause 1, further comprising: a support ring disposed within the tubing and aligned with the tubing assembly connector, the support ring having a support ring modulus greater than the tubing elastic modulus.

Clause 13. A method, comprising: abutting a component connector of a tubing system component against a tubing assembly connector of a tubing assembly to permit fluid communication between the tubing system component and the tubing assembly; and
overmolding a coupling portion over the tubing assembly connector and the component connector to couple the tubing assembly connector and the component connector.

Clause 14. The method of Clause 13, further comprising: overmolding the tubing assembly connector over a tubing of the tubing assembly before abutting the component connector against the tubing assembly connector.

Clause 15. The method of Clause 14, further comprising: inserting a core pin into a lumen of the tubing prior to overmolding the tubing assembly connector.

Clause 16. The method of Clause 15, further comprising: removing the core pin from the lumen of the tubing after overmolding the tubing assembly connector.

Clause 17. The method of Clause 14, further comprising: inserting a support ring into a lumen of the tubing prior to overmolding the tubing assembly connector.

Clause 18. A method, comprising: abutting a component connector of a tubing system component against a tubing assembly connector of a tubing assembly to permit fluid communication between the tubing system component and the tubing assembly; and ultrasonically welding the component connector and the tubing assembly connector to couple the tubing assembly connector and the component connector.

Clause 19. The method of Clause 18, further comprising: overmolding the tubing assembly connector over a tubing of the tubing assembly before abutting the component connector against the tubing assembly connector.

Clause 20. The method of Clause 18, wherein the tubing assembly connector comprises a tapered interface to engage with the component connector.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

In one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Various items may be arranged differently (e.g., arranged in a different order, or partitioned in a different way) all without departing from the scope of the subject technology. All structural and functional equivalents to the elements of the various aspects described throughout this disclosure that are known or later come to be known to those of ordinary skill in the art are expressly incorporated herein by reference and are intended to be encompassed by the claims. Moreover, nothing disclosed herein is intended to be dedicated to the public regardless of whether such disclosure is explicitly recited in the claims. No claim element is to be construed under the provisions of 35 U.S.C. §112, sixth paragraph, unless the element is expressly recited using the phrase "means for" or, in the case of a method claim, the element is recited using the phrase "step for." Furthermore, to the extent that the term "include," "have," or the like is used, such term is intended to be inclusive in a manner similar to the term "comprise" as "comprise" is interpreted when employed as a transitional word in a claim.

The Title, Background, Summary, Brief Description of the Drawings and Abstract of the disclosure are hereby incorporated into the disclosure and are provided as illustrative examples of the disclosure, not as restrictive descriptions. It is submitted with the understanding that they will not be used to limit the scope or meaning of the claims. In addition, in the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or operation. The following claims are hereby incorporated into the Detailed Description, with each claim standing on its own as a separately claimed subject matter.

The claims are not intended to be limited to the aspects described herein, but is to be accorded the full scope consistent with the language claims and to encompass all legal equivalents. Notwithstanding, none of the claims are intended to embrace subject matter that fails to satisfy the requirement of 35 U.S.C. §101, 102, or 103, nor should they be interpreted in such a way.

## Claims

1. A method, comprising:
providing a tubing assembly (150) comprising a tubing (152) and a tubing assembly connector (158) circumferentially disposed around a portion of the tubing;
providing a tubing system component comprising a component connector (132);
disposing a portion of the tubing assembly connector within the component connector;
disposing a support ring (270) within a lumen (151) of the tubing; and
molding a coupling portion (160) over the tubing assembly connector and the component connector.

2. The method of Claim 1, wherein providing the tubing assembly comprises molding the tubing assembly connector over the portion of the tubing.

3. The method of Claim 1, wherein providing the tubing assembly comprises providing the tubing and the tubing assembly connector such that the tubing has a first elastic modulus that is less than a second elastic modulus of the tubing assembly connector.

4. The method of Claim 1, wherein providing the tubing system component comprises providing the component connector such that the component connector has a third elastic modulus that is greater than the first elastic modulus.

5. The method of Claim 1, wherein disposing the portion of the tubing assembly connector within the component connector further comprises ultrasonically welding the component connector and the tubing assembly connector.

6. The method of Claim 1, wherein disposing the support ring within the lumen of the tubing comprises providing the support ring such that the support ring has a fourth elastic modulus that is greater than the first elastic modulus.

7. The method of Claim 1, wherein molding the coupling portion over the tubing assembly connector and the component connector comprises circumferentially disposing the coupling portion around the tubing assembly connector and the component connector.

8. The method of Claim 1, wherein molding the coupling portion over the tubing assembly connector and the component connector comprises applying a pressure to the tubing assembly connector and the component connector, and the pressure is between 5,000 pounds per square inch (psi) and 12,000 psi.

9. The method of Claim 1, further comprising:
disposing a core pin within a lumen of the tubing.

10. The method of Claim 9, further comprising:
removing the core pin from the lumen of the tubing.

11. The method of Claim 1, further comprising:
providing a check valve (380) comprising a check valve connector (382); and
coupling the check valve connector to the tubing assembly connector.

12. A method, comprising:
providing a tubing assembly (150) comprising a tubing (152) and a tubing assembly connector (158) circumferentially disposed around a portion of the tubing;
providing a check valve (380) comprising a check valve connector (382);
disposing a portion of the tubing assembly connector within the check valve connector; and
molding a coupling portion (160) over the tubing assembly connector and the check valve connector.

13. The method of Claim 12, wherein providing the tubing assembly comprises molding the tubing assembly connector over the portion of the tubing.

14. The method of Claim 12, wherein molding the coupling portion over the tubing assembly connector and the component connector comprises applying a pressure to the tubing assembly connector and the component connector, and the pressure is between 5,000 pounds per square inch (psi) and 12,000 psi.
